Europäisches Patentamt

European Patent Office

Office européen des brevets

(19)

(11) Publication number: **0 546 176 A1**

(12) **EUROPEAN PATENT APPLICATION**
published in accordance with Art. 158(3) EPC

(21) Application number: 91911956.0

(22) Date of filing: 27.06.91

(86) International application number:
PCT/JP91/00876

(87) International publication number:
WO 93/00428 (07.01.93 93/02)

(51) Int. Cl.⁵: **C12N 15/10, C12N 15/11**

(43) Date of publication of application:
16.06.93 Bulletin 93/24

(84) Designated Contracting States:
AT BE CH DE DK ES FR GB IT LI LU NL SE

(71) Applicant: SUMITOMO DENKI KOGYO KABUSHIKI KAISHA
5-33, Kitahama 4-chome, Chuo-ku
Osaka-shi, Osaka 541(JP)

(72) Inventor: MATSUSHIRO, Aizo, 15-12,
Aoyamadai 4-chome
Suita-shi
Osaka 565(JP)
Inventor: MORITA, Takashi, 4-33,
Uenocho-Higashi
Kishiwada-shi
Osaka 596(JP)

(54) NOVEL DNA PREPARATION PROCESS AND PRIMER USED FOR DNA PREPARATION.

(57) A novel DNA preparation process which comprises preparing a chain DNA (A) composed of a number of deoxyribonucleotides and a primer for the DNA (A), preferably one having a base sequence of TCCAGCGGCGGG or AGGTCGCCGCCC, annealing a single-stranded DNA (D) prepared by thermally altering the DNA (A) with the primer to form a chain DNA (E), synthesizing a complementary chain (F) from the DNA (E) and DNA polymerase to prepare a double-stranded DNA (X), and replicating the DNA (X) by repeating the steps of the thermal alteration, the annealing with the primer, and the synthesis of the complementary chain.

Fig.1

(Technical Field)

The present invention relates to a method for preparing DNA, particularly to a method for preparing long-chain DNA, and a novel primer for preparing DNA.

(Background Art)

According to the development of recent years on the technology of the gene manipulation, the search of the unknown useful DNA and to prepare an artificial DNA are come to be popular.

With regard to the means for artificially preparing DNA above, there had been known the method for preparing a chain DNA by linking a molecule of the deoxyribonucleotide one by one with the reagent.

However, in the conventional method for preparing DNA and the devices therefor, there had been pointed out the following problems;

(1) The yields of DNA to be prepared in a linking-reaction of the molecule of the deoxyribonucleotide one by one would be cumulatively reduced as the chain becomes longer, therefore, a difficulty in obtaining the chain DNA having the desired base sequences comes to be increased as the length of the chain becomes longer.

(2) In connection with (1) above, when the enough amounts of the chain DNA being comparatively long is prepared, a remarkable amounts of DNA being different from the desired DNA are by-produced, thereby the considerable amounts of the reagent are consumed additionaly, most of the reagent used are discarded at the time of completing the preparation of the chain DNA, and the expensive cost on the reagent is only remainded.

(3) Since about 10 minutes would be required to link one nucleotide, the time for preparing the chain DNA being comparatively long becomes long, thereby a considerable labor burden have to be needed.

(4) Further, a conventional device for preparing DNA can only synthesize the single stranded DNA.

Due to the problems of the prior art, although the conventional device for preparing DNA was conveninet to prepare the chain DNA being comparatively short (about 30-50bp), a realization by the conventional device of the preparation of the chain DNA (of several hundred bases or more) of the gene existing in the nature and requiring at present in the research was difficult, because there were problems on the time and the labor burden respectively required in the preparing procedure, little yields of the target DNA, and an economical subject of discarding in vain the most of the reagent used in the preparing procedure.

(Disclosure of the Invention)

The present invention was invented in view of the subject above and for a purpose of providing the large amounts of the desired long chain DNA in a short time by recognizing a trace of long chain DNA artificially prepared in the construction step and selectively replicating it in the replication step added to the conventional method for preparing DNA.

The present invention is a method for preparing DNA comprising the series steps of:

(a) preparing a chain DNA comprising the plural deoxyribonucleotide linked with the bonding agent, and a primer of said chain DNA,

(b) conducting a thermal denaturation of said chain DNA,

(c) annealing said primer to said chain DNA denaturated thermally,

(d) synthesizing a complementary chain by using the chain DNA annealed said primer thereto and DNA polymerase, and preparing a double stranded DNA, and

(e) replicating said double stranded DNA by repeating the foregoing steps (b), (c) and (d).

(Brief Description of the Drawings)

Figure 1 is a schematic view showing a replication step of the present invention;

Figure 2 is a schematic view showing a thermal control condition (temperature and time) of the replication step of the present invention; and

Figure 3 is an illustration of the electrophoresis using, Molecular Weight Marker $\phi \times$ 174 HaeIII fragment (Y),

unreplicated 1 pg/$\mu$l Template DNA (1),

replicated 1 pg/$\mu$l Template DNA (2),

unreplicated 50pg/$\mu$l Template DNA (3),

replicated 50pg/μl Template DNA (4),
unreplicated 1 ng/μl Template DNA (5),
replicated 1 ng/μl Template DNA (6),
unreplicated 50ng/μl Template DNA (7),
replicated 50ng/μl Template DNA (8),
unreplicated 1 μg/μl Template DNA (9), and
replicated 1 μg/μl Template DNA (10).

(Best Modes for Carrying Out the Invention)

A method for preparing DNA of the present invention will be described in detail in the following by referring to the drawings.

First of all, a construction step for preparing a single stranded DNA and a primer of said single stranded DNA is described in the following.

① To input an information of the base sequence of the target single stranded DNA into the program board of the device (Trade Name "Model 381A DNA Synthesizer", Applied Biosystems) for preparing DNA.

② The target single stranded DNA is constructed by succesively releasing, in accordance with the inputted program, the bases of every kind of constituting DNA respectively held in the plural Phospho Amidide Bottle and the bonding agent (comprising Trichloroacetic Acid, Tetrazole and $H_2$ $O/I_2$ ) for bonding DNA molecular into the synthesis-column mounted on the device for preparing DNA, and linking the nucleotide one by one with the chemical reaction.

③ The primer of said single stranded DNA is also constructed according to said method.

The impurity produced at the time of synthesizing the chain DNA contains DNA being shorter than the target chain DNA stopped halfway the extension started from 3' terminal of the chain DNA to 5' terminal.

Therefore, to shorten the complementary portion (to be annealed to the chain DNA) of the primer at 5' terminal side of the chain DNA is preferrable to specifically and effectively conduct the amplification of the target single stranded DNA.

Further, in order to effectively synthesize in a short time the single stranded DNA and the primer for obtaining the target long chain DNA, 5' and 3' terminal of the target long chain DNA have to be determined by the primer at 5' and 3' terminal side respectively and the single stranded DNA also have to be synthesized within the sequence of the target stranded DNA. For example, the single stranded DNA to be prepared have the same length with that of the target stranded DNA, and 5' and 3' terminal side of the primer to be used in the preparing procrdure respectively initiate the synthesis of DNA regarding 5' and 3' terminal of the target chain DNA as 5' terminal of the each primer.

To use the primer incorporated much bases of AT or GC therein is more preferrably for enhancing the specificity of the primer for the target stranded DNA. However, the primer having the sequence aforementioned can not be actually used because said primer itself tends to have a secondary structure. The present inventors have directed the attention to λ phage of having a portion of the base pairs (TCCAGCGGCG GG) set out in SEQ ID NO: 1 incorporated a lot of bases GC therein as a portion of the cohesive end, and acquire a knowledge therefrom of showing a remarkable specificity between the primer having 12 base pairs above and the target DNA at the time of annealing. Further, when two types of primers respectively corresponding to 5' and 3' terminal are required, the specificity can be enhanced by using the sequence (AGGTCGC CGCCC) set out in SEQ ID NO: 2 being completely reverse to said base pairs.

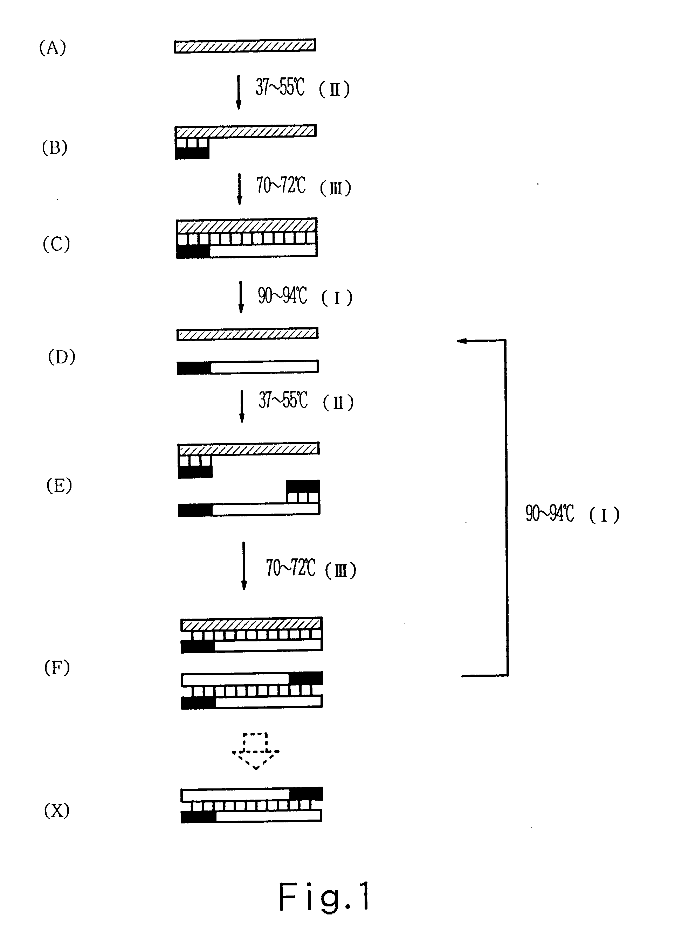

The replication step of the single stranded DNA prepared in the construction step is described in the following along with Fig. 1.

④ The primer is annealed to one edge of the single stranded DNA (the shaded area is DNA portion to be needed) (A) prepared in the construction step by transferring said single stranded DNA into the tube, mounting the tube on the device (Trade Name "BiGene PHC-1", Techne) for replicating DNA, and maintainig the temperature (of water) 37-55 °C for 2 minutes.

⑤ The complementary chain (C) is synthesized from said annealed single stranded DNA (B) with the thermal resistant polymerase by maintainig the temperature 70-72°C for 3 minutes.

⑥ Said complementary chain (C) is reconstructed into the single stranded DNA (D) by maintainig the temperature 90-94°C for 1 minute and thermally denaturalizing.

⑦ The primers are annealed by maintaining the temperature 37-55°C for 2 minutes to the both edges of the single stranded DNA (D) obtained by said thermal denaturation.

⑧ The complementary chain (F) is synthesized from said annealed single stranded DNA (E) with the thermal resistant polymerase by maintainig the temperature 70-72°C for 3 minutes.

⑨ The desired amounts of the double stranded DNA fragment (X) is prepared by repeating the foregoing series steps of ⑥~⑧ appropriately and replicating (amplifying) selectively.

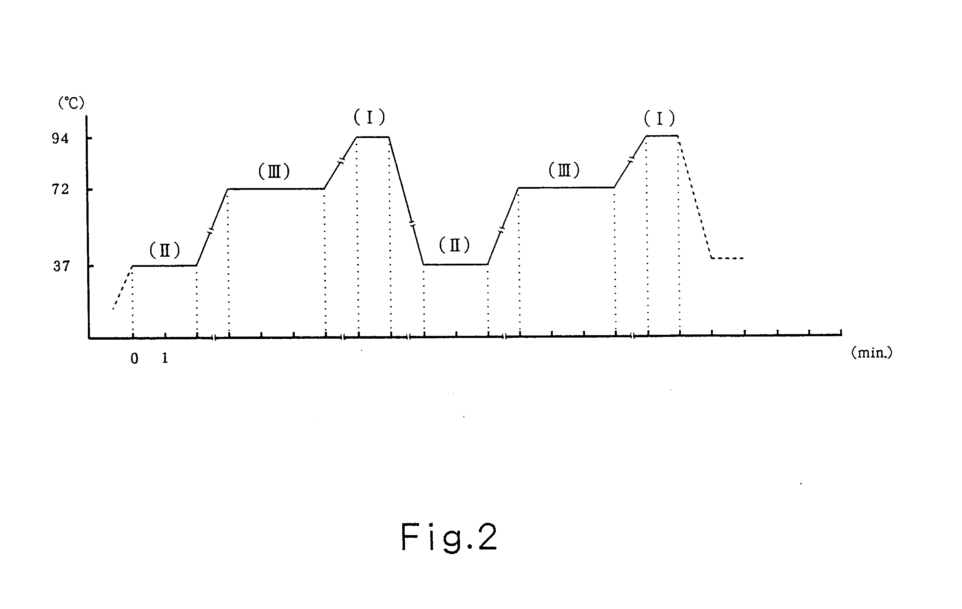

The relation between the time and the temperature for smoothly conducting the reactions ⑥~⑧ of said replication step is shown in Fig. 2. It was acknowledged that the preferable relation between the time and the temperature for completing each reaction are 90-94 °C and 1 minute for the thermal denaturation (I), 37-55°C and 2 minutes for annealing of the primer (II), and 70-72 °C and 3 minutes for synthesizing the complementary chain (III).

Example 1: Preparation of Reagent to be used in Replication Step

The following reagents were prepared.
(1) Thermal Resistant Polymerase
Taq polymerase (Cetus)
(2) d-ATP, d-CTP, d-GTP, d-TTP
(3) Buffer (concentrated to be used as a tenfolds dilution)
(4) oligonucleotide primer MM1 (SEQ ID No. 3: 25 mer), and oligonucleotide primer MM2 (SEQ ID No. 4: 25 mer)
An oligonucleotide primer prepared by the conventional construction step and being complementary with each side of 3' terminal of the target double stranded DNA portion.
(5) Template DNA: Single stranded DNA (SEQ ID No. 5: 140 bases) prepared according to the conventional construction step. A trace of Template DNA may be accceptable because it recognizes the existence of the several molecules of the target DNA and replicates it selectively.

Example 2: Mixing of Reaction Solution

The reagents prepared in the Example 1 were mixed in accordance with the order described in the following Table 1. Attention on the mixing order of the reagent should be paid.

Table 1

| | |
|---|---|
| (1) Distilled Water | 70 $\mu$l |
| (2) Buffer of tenfolds dilution | 10 $\mu$l |
| (3) d-NTP (200 $\mu$M)(2 $\mu$l ×4 types) | 8 $\mu$l |
| (4) oligonucleotide primer (1 $\mu$M) (5 $\mu$l ×2 types) | 10 $\mu$l |
| (5) Template DNA (trace) | 1 $\mu$l |
| (6) Thermal Resistant DNA Polymerase Taq polymerase (5000U/ml) | 1 $\mu$l |
| Total | 100 $\mu$l |

To prevent the change of the composition of the solution by an evaporation of the solution, the mineral oil or the liquid paraffin was applied thereto.

Example 3: Illustration of Experiment

An experiment on the replication of DNA was conducted by using the reagents listed in the following Table 2 and the device (Trade Name "BiGene PHC-1", Techne) for replicating DNA.

(1) Mixing of the Reagents

The reagents listed in the following Table 2 were added to the five tubes respectively in accordance with the order of the list. The identical amounts of the reagents, except for Template DNA, was added to all tubes.

4

Table 2

| | | |
|---|---|---|
| (1) Distilled Water | | 70 $\mu$l |
| (2) Buffer of tenfolds dilution | | 10 $\mu$l |
| (3) d-ATP (10mM) | | 2 $\mu$l |
| (4) d-CTP (10mM) | | 2 $\mu$l |
| (5) d-GTP (10mM) | | 2 $\mu$l |
| (6) d-TTP (10mM) | | 2 $\mu$l |
| (7) oligonucleotide primer MM1 (25mer, 20 $\mu$M) | | 5 $\mu$l |
| (8) oligonucleotide primer MM2 (25mer, 20 $\mu$M) | | 5 $\mu$l |
| (9) Template DNA | | |
| Tube (No.1) | Template DNA (1 $\mu$g/ $\mu$l) | 1 $\mu$l |
| Tube (No.2) | Template DNA (50ng/ $\mu$l) | 1 $\mu$l |
| Tube (No.3) | Template DNA (1ng/ $\mu$l) | 1 $\mu$l |
| Tube (No.4) | Template DNA (50pg/ $\mu$l) | 1 $\mu$l |
| Tube (No.5) | Template DNA ( 1pg/ $\mu$l) | 1 $\mu$l |
| (10) Taq polymerase (5000U/ml) | | 1 $\mu$l |
| (11) Liquid Paraffin | | 20 $\mu$l |
| Total | | 120 $\mu$l |

(2) Thermal Control

An adjustments of said reaction temperature would be required about 1 minute, therefore, the condition was set 94°C/2 minutes for the thermal denaturation, 37°C/3 minutes for annealing of the primer, and 72°C/4 minutes for synthesizing the complementary chain.

(3) Results

Having completed the 20 cycles of the reaction, 20 $\mu$l (1/5 volume) of the reaction solution 100 $\mu$l was applied to 8% polyacrylamide gel electrophoresis.

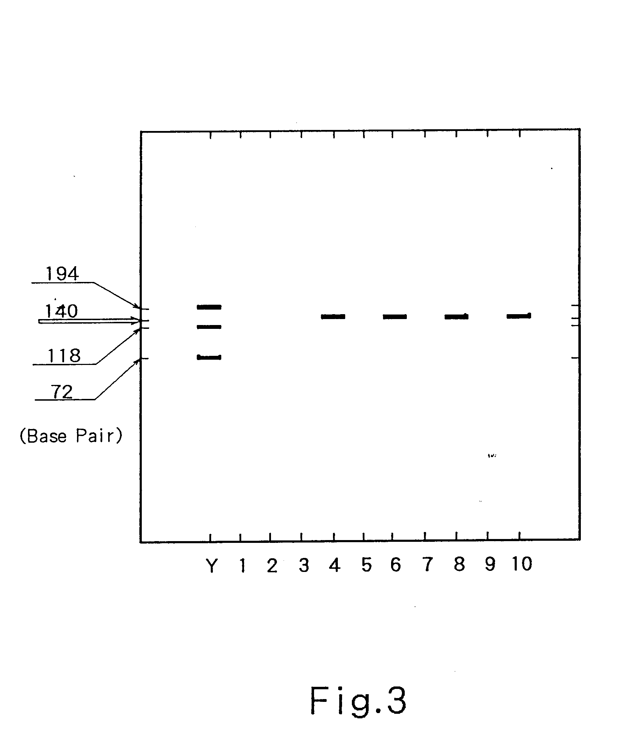

The results of the electrophoresis were shown in Fig. 3.

As a result of Fig. 3, an existence of DNA did not confirm in the lane (Fig. 3: lane 9) of Template DNA concentration 1 $\mu$g/ $\mu$l being maximum within the lane (Fig. 3: odd number lanes) used the various concentration of the unreplicated Template DNA, in contrast thereto, there had been confirmed the replicated band (See white arrow of Fig. 3) of DNA having 140 base pairs in the lane (Fig. 3: lane 4) of the thin Template DNA concentration 50pg/ $\mu$l within the lane (Fig. 3: even number lanes) used the various concentration of the Template DNA prepared (replicated) in accordance with the method of the present invention, thereby it can be confirmed that the replication step of the present invention can recognaize the trace DNA and selectively replicate it.

(Industrial Applicability)

The present invention provides, by adding the replication step to the conventional method for preparing DNA, the large amounts of the desired long chain DNA in a short time from a trace of the long chain DNA prepared in the construction step.

According to the present invention, by adding the replication step to the conventional method for preparing DNA, the large amounts of the double stranded DNA, being long chain DNA obtained conventionally only directly from an animal and a plant of the nature and having the several hundred of the bases or more, can be artificialy synthesized from an extremely trace of the long chain DNA prepared in the construction step. Further, by the present invention, to regenerate and replicate the desired chain DNA based on the known information (e.g., base sequences) come to be possible.

Since, in principle, the replication step of the present invention make use of the complementarity of the bases, in addition to DNA, RNA can also be applied thereto.

Further, as an useful method for using a long chain DNA prepared by the present invention, a method for producing the novel peptides or the enzymes by introducing the prepared DNA into a bacteria such as Escherichia coli can be expected.

As referred to the above, according to the method for producing DNA of the present invention, the long chain genetic DNA, which an animal and a plant of the nature may have, can be artificially synthesized, regenerated and replicated, therefore, the contribution to the field of the industry and the science as the means for optionaly generating the novel material or the novel organism can well be expected.

(Sequence Listing)

SEQ ID No. : 1

Sequence Length: 12

Sequence Type: nucleic acid

Strandedness : single

Topology : Linear

Molecule Type:  Other nucleic acid,  Synthetic DNA

Sequence Description

TCCAᵇCGGCG GG                                                                    12


SEQ ID No. : 2

Sequence Length: 12

Sequence Type: nucleic acid

Strandedness : single

Topology : Linear

Molecule Type:  Other nucleic acid,  Synthetic DNA

Hypothetical Sequence: Yes

Sequence Description

AGGTCGCCGC CC                                                                    12


SEQ ID No. : 3

Sequence Length: 25

Sequence Type: nucleic acid

Strandedness : single

Topology : Linear

Molecule Type:  Other nucleic acid,  Synthetic DNA

Hypothetical Sequence: Yes

Sequence Description

TCTCCTGTGG CAGATGCCCC AAATA 25

SEQ ID No. : 4

Sequence Length: 25

Sequence Type: nucleic acid

Strandedness : single

Topology : Linear

Molecule Type:  Other nucleic acid,  Synthetic DNA

Hypothetical Sequence: Yes

Sequence Description

CATGACCAGA TGTCGAGCTG AGAAC 25

SEQ 1D No. : 5

Sequence Length: 140

Sequence Type: nucleic acid

Strandedness : single

Topology : Linear

Molecule Type:  Other nucleic acid,  Synthetic DNA

Hypothetical Sequence: Yes

Sequence Description

TCTCCTGTGG CAGATGCCCC AAATAGGATC CGACCCTAGC 40

AGCGCCACCG TGGGGCCCAA GGCGCCGGCG GTCGGCAAGA 80

AGGCCTCGCA GGCCTCTCAG GACTACTGAA AGCTTGTTCT 120

CAGCTCGAAC TCTGGTCATG 140

## Claims

1. A method for preparing DNA comprising the steps of:
   (a) preparing a chain DNA comprising the plural deoxyribonucleotide linked with a bonding agent, and a primer of said chain DNA,
   (b) conducting a thermal denaturation of said chain DNA,
   (c) annealing said primer to said chain DNA denatured thermally,
   (d) synthesizing a complementary chain by using the chain DNA annealed said primer thereto and DNA polymerase, and preparing double stranded DNA, and
   (e) replicating said double stranded DNA by repeating the foregoing series steps (b), (c) and (d).

8

2. A method according to Claim 1, wherein said chain DNA is a single stranded DNA.

3. A method according to Claim 2, wherein said single stranded DNA comprising DNA molecule having one hundred basees or more.

4. A method according to Claim 1, wherein the both terminals of said chain DNA is annealed to become 5' terminal of the primer.

5. A method according to Claim 1, wherein said primer comprising the base sequence of TCCAGCGGCGGG.

6. A method according to Claim 1, wherein said primer comprising the base sequence of AGGTCGCCGCCC.

7. A primer for preparing DNA comprising the base sequence of TCCAGCGGCGGG.

8. A primer for preparing DNA comprising the base sequence of AGGTCGCCGCCC.

(A)

↓ 37~55℃ （Ⅱ）

(B)

↓ 70~72℃ （Ⅲ）

(C)

↓ 90~94℃ （Ⅰ）

(D)

↓ 37~55℃ （Ⅱ）

(E)

90~94℃ （Ⅰ）

↓ 70~72℃ （Ⅲ）

(F)

(X)

# Fig.1

Fig.2

EP 0 546 176 A1

Fig.3

# INTERNATIONAL SEARCH REPORT

International Application No PCT/JP91/00876

## I. CLASSIFICATION OF SUBJECT MATTER (if several classification symbols apply, indicate all) 6

According to International Patent Classification (IPC) or to both National Classification and IPC

Int. Cl⁵ C12N15/10, C12N15/11

## II. FIELDS SEARCHED

### Minimum Documentation Searched 7

| Classification System | Classification Symbols |
|---|---|
| IPC | C12N15/00-15/90 |

### Documentation Searched other than Minimum Documentation to the Extent that such Documents are Included in the Fields Searched 8

Biosis Database, Chemical Abstract Database (CA, REG)

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| Y | JP, A, 62-000281 (F. Hoffmann-La Roche AG.), January 6, 1987 (06. 01. 87), (Family: none) | 1-8 |
| Y | JP, A, 61-274697 (F. Hoffmann-La Roche AG.), December 4, 1986 (04. 12. 86), (Family: none) | 1-8 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| March 25, 1992 (25. 03. 92) | April 7, 1992 (07. 04. 92) |

| International Searching Authority | Signature of Authorized Officer |
|---|---|
| Japanese Patent Office | |

Form PCT/ISA/210 (second sheet) (January 1985)